(19) Europäisches Patentamt
European Patent Office
Office européen des brevets

(11) **EP 2 857 065 B1**

(12) **FASCICULE DE BREVET EUROPEEN**

(45) Date de publication et mention
de la délivrance du brevet:
**04.05.2016 Bulletin 2016/18**

(51) Int Cl.:
*A61N 1/378* *(2006.01)*        *H02N 2/18* *(2006.01)*
*H02N 11/00* *(2006.01)*        *H01L 41/053* *(2006.01)*
*A61N 1/375* *(2006.01)*        *H02K 35/02* *(2006.01)*
*H02N 1/08* *(2006.01)*

(21) Numéro de dépôt: **14180560.6**

(22) Date de dépôt: **11.08.2014**

(54) **Capsule intracorporelle autonome à récupération d'énergie avec conversion de fréquence**

Autonome intrakorporelle Kapsel mit Energierückgewinnung über Frequenzwandlung

Autonomous intracorporeal capsule having energy recovery with frequency conversion

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **01.10.2013 FR 1359466**

(43) Date de publication de la demande:
**08.04.2015 Bulletin 2015/15**

(73) Titulaire: **Sorin CRM SAS**
**92140 Clamart Cedex (FR)**

(72) Inventeurs:
• **Deterre, Martin**
**75013 Paris (FR)**
• **Lefeuvre, Elie**
**93100 Montreuil (FR)**

(74) Mandataire: **Dupuis-Latour, Dominique**
**Bardehle Pagenberg**
**10, boulevard Haussmann**
**75009 Paris (FR)**

(56) Documents cités:
EP-A1- 2 520 333        EP-A1- 2 638 930
EP-A1- 2 639 845        DE-A1-102004 043 002
US-A1- 2007 276 444        US-A1- 2011 140 577

## Description

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les implants cochléaires, etc., ainsi que les dispositifs de mesure de pH ou encore d'impédance intracorporelle (telle que mesure d'impédance transpulmonaire ou d'impédance intracardiaque).

L'invention concerne plus particulièrement ceux de ces dispositifs qui mettent en oeuvre des capsules autonomes implantées et dépourvues de toute liaison physique à un dispositif principal implanté (tel qu'un boitier de générateur d'impulsions de stimulation).

Ces capsules autonomes sont dénommées pour cette raison "capsules *leadless*", pour les distinguer des électrodes ou des capteurs disposés à l'extrémité distale d'une sonde (*lead*), cette sonde étant parcourue sur toute sa longueur par un ou plusieurs conducteurs reliant par voie galvanique l'électrode ou le capteur à un générateur connecté à l'extrémité opposée, proximale, de la sonde.

De telles capsules *leadless* sont par exemple décrites dans les US 2007/0088397 A1 et WO 2007/047681 A2 (Nanostim, Inc.) ou encore dans le US 2006/0136004 A1 (EBR Systems, Inc.).

Ces capsules *leadless* peuvent être par exemple des capsules épicardiques, fixées à la paroi extérieure du coeur, ou bien des capsules endocavitaires, fixées à la paroi intérieure d'une cavité ventriculaire ou auriculaire au moyen d'une vis d'ancrage saillante, prolongeant axialement le corps de la capsule et destinée à pénétrer dans le tissu cardiaque par vissage au site d'implantation. L'invention n'est toutefois pas limitée à un type particulier de capsule, et elle est applicable indifféremment à tout type de capsule *leadless,* quelle que soit sa destination fonctionnelle.

Une capsule *leadless* comprend divers circuits électroniques, capteurs, etc. ainsi que des moyens émetteurs/récepteurs de communication sans fil pour l'échange de données à distance.

Dans tous les cas, le traitement des signaux au sein de la capsule et leur transmission à distance nécessite une énergie non négligeable par rapport aux ressources énergétiques que peut stocker cette capsule. Or, compte tenu de son caractère autonome, la capsule ne peut faire appel qu'à ses ressources propres, à savoir un système d'auto-alimentation intégré comprenant un récupérateur d'énergie associé à une petite batterie tampon intégrée.

Un premier type de récupérateur d'énergie utilise un transducteur couplé à un mécanisme inertiel comportant une masse mobile, dite "masse sismique", oscillant dans la capsule au gré des déplacements de cette dernière, qui est soumise aux efforts dus aux mouvements de la paroi de l'organe du patient et aux forces fluidiques du milieu environnant. La puissance récupérée dépend principalement de la fréquence d'excitation de la masse sismique, de l'amplitude de ce mouvement et de la valeur de masse. Or, dans le cas de l'environnement du corps humain, les excitations provenant de l'accélération du corps ou des organes n'ont pas de fréquences spécifiques stables pour lesquelles la récupération pourrait être optimisée de manière à produire une résonance du mécanisme. De la sorte, il n'est pas possible de bénéficier d'une amplification mécanique qui accroitrait l'amplitude et permettrait de récupérer un maximum de l'énergie inertielle. De plus, les fréquences d'excitation en jeu sont très basses, de l'ordre de 0,5 à 10 Hz pour les fréquences typiques de pulsation du flux sanguin et de 15 à 40 Hz pour les mouvements des parois cardiaques, ce qui limite les performances du récupérateur. Enfin, la valeur de masse de la masse sismique doit rester très faible, pour des raisons de respect de la miniaturisation.

Un autre type de récupérateur d'énergie, non inertiel, utilise les variations de pression du fluide environnant la capsule (typiquement le milieu sanguin) pour déformer ou déplacer cycliquement une membrane flexible ou un soufflet couplé à un transducteur. L'énergie susceptible d'être récupérée dépend principalement de l'amplitude du mouvement cyclique, de la membrane ou du soufflet actionné par le fluide environnant (amplitude qui est nécessairement limitée pour des raisons de fiabilité mécanique), de la fréquence de ce mouvement cyclique et de l'aire de la surface mobile (nécessairement réduite pour des raisons évidentes de miniaturisation de la capsule). Ici encore, les variations de pression se situent à la fréquence cardiaque, de l'ordre de 1 à 3 Hz, et ne permettent donc de solliciter le transducteur qu'à basse fréquence, imposant de fait une limitation des performances du récupérateur d'énergie.

Le but de l'invention est de pallier ces limitations en proposant un nouveau type de récupérateur d'énergie pourvu d'un mécanisme permettant d'accroitre la fréquence d'excitation du transducteur, de manière à bénéficier, pour un seul cycle de sollicitation externe, d'une pluralité de cycles de transduction pour la conversion de l'énergie mécanique procurée.

Sur cet aspect, le US 2011/0140577 A1 décrit un dispositif de récupération d'énergie vibratile comprenant deux aimants suspendus, montés en vis-à-vis et en opposition de pôles et portés chacun par une membrane élastique, avec entre eux une masse inertielle portant un troisième aimant intercalaire. Le va-et-vient de la masse inertielle provoque des couplages/découplages successifs des aimants suspendus, à une fréquence supérieure à celle de l'oscillation de la masse inertielle. L'énergie d'oscillation de chaque aimant suspendu est récupérée par un bobine fixe à l'intérieur de laquelle oscille l'aimant. Cette structure permet certes d'améliorer le rendement de récupération de l'énergie, mais elle conserve toutefois les inconvénients exposés plus haut tenant à la double présence, par nature indispensable, d'une masse sismique et de moyens magnétiques.

Le document EP 2 520 333 décrit une capsule intracorporelle autonome selon le préambule de la revendication 1.

Un autre but de l'invention est de proposer un tel mécanisme qui puisse être utilisé aussi bien avec un récupérateur inertiel à masse sismique, sollicité par les vibrations et mouvements externes du milieu environnant, qu'avec un récupérateur non inertiel de type à membrane ou à soufflet, sollicité par les variations cycliques de pression du fluide dans lequel baigne la capsule.

Un autre but encore de l'invention est de proposer un tel mécanisme qui ne mette en oeuvre ni élément magnétique qui créerait un risque lors d'un examen IRM, ni choc ou contact mécanique répétitif qui entrainerait sur le long terme des problèmes de fiabilité mécanique.

L'idée de base de l'invention consiste à mettre en oeuvre deux éléments mobiles, à savoir :

- une première structure, ci-après "structure oscillante primaire" soumise à une sollicitation extérieure à basse fréquence qui peut être une force directement appliquée par exemple à partir des variations de pression déplaçant cycliquement une membrane ou un soufflet, ou bien indirectement par une masse sismique solidaire de cette structure, cette structure se déplaçant à cette même fréquence basse ; et
- une deuxième structure, ci-après "structure oscillante secondaire" vibrant à plus haute fréquence, typiquement avec un effet de résonance et couplée au transducteur du récupérateur d'énergie.

Pour réaliser cette conversion de fréquence, les deux structures primaire et secondaire sont couplées entre elles par un mécanisme réalisant une fonction de "tirer-lâcher" créant un phénomène de relaxation.

Ce couplage entre les deux structures oscillantes est réalisé, de façon caractéristique, par une structure de couplage opérant par interaction électrostatique, donc sans élément magnétique ni élément mécanique frottant ou à impact.

Plus précisément, l'invention propose une capsule intracorporelle autonome comportant, de manière en elle-même connue, un corps et, à l'intérieur de ce corps, des circuits électroniques et un module de récupération d'énergie pour l'alimentation électrique de ces circuits électroniques. Le module de récupération d'énergie comprend un transducteur apte à convertir en énergie électrique une sollicitation physique extérieure cyclique appliquée au corps de la capsule et résultant de variations de pression dans le milieu environnant la capsule et/ou de mouvements d'une paroi à laquelle est ancrée la capsule.

Le module de récupération d'énergie comprend :

- une structure oscillante primaire soumise à la sollicitation cyclique extérieure, cette structure oscillante primaire étant apte à être déplacée alternativement dans un sens et dans l'autre à la fréquence de la sollicitation cyclique extérieure ;
- une structure oscillante secondaire non soumise à la sollicitation extérieure cyclique, cette structure oscillante secondaire comprenant un élément élastique déformable et étant apte à vibrer librement à une fréquence de résonance propre supérieure à la fréquence de la sollicitation cyclique extérieure ; et
- une structure électrostatique comprenant une première électrode de condensateur couplée à la structure oscillante primaire ou au corps de la capsule, et une seconde électrode de condensateur couplée à la structure oscillante secondaire.

La première et la seconde électrode sont configurées entre elles de manière que, sous l'effet de la sollicitation cyclique extérieure appliquée à la structure oscillante primaire, elles exercent entre elles sous l'effet d'interactions électrostatiques une force d'attraction mutuelle entrainant la structure oscillante secondaire en éloignement de sa position d'équilibre stable et avec mise en tension de l'élément élastique déformable. La structure oscillante secondaire est ainsi entrainée jusqu'à une limite où la tension exercée par l'élément élastique déformable dépasse la force d'attraction mutuelle des électrodes, limite au-delà de laquelle la structure oscillante secondaire est libérée par effet de relaxation pour vibrer librement à ladite fréquence de résonance propre. Enfin, le transducteur est couplé à la structure oscillante secondaire, pour convertir en énergie électrique les mouvements de vibration de celle-ci à ladite fréquence de résonance propre.

Le transducteur peut être de type électrostatique. Il comprend alors des électrodes de conversion formant un condensateur, l'une de ces électrodes de conversion étant entrainée cycliquement par la structure oscillante secondaire, et le transducteur opérant par modification cyclique des surfaces en vis-à-vis et/ou de l'intervalle diélectrique des électrodes de conversion avec variation corrélative de la capacité du condensateur. Les électrodes de conversion peuvent notamment inclure les première et seconde électrodes de la structure électrostatique.

De façon caractéristique de l'invention, la structure oscillante primaire et la structure oscillante secondaire sont couplées entre elles par l'élément élastique déformable. La première électrode de la structure électrostatique est reliée au corps de la capsule, la seconde électrode de la structure électrostatique étant reliée à la structure oscillante secondaire.

Les revendications dépendantes définissent des modes de réalisation préférés de l'invention.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 illustre de façon schématique un ensemble de dispositifs médicaux comprenant notamment des capsules *leadless*, implantées au sein du corps d'un patient.

La Figure 2 est un schéma par blocs fonctionnels montrant les différents étages constitutifs d'une capsule *leadless.*

Les Figures 3 et 4 illustrent deux formes de réalisation possibles du corps d'une capsule *leadless* à récupération des variations de pression du fluide environnant.

Les Figures 5 à 7 illustrent diverses variantes de la structure interne d'une capsule *leadless* connue à récupération des variations de pression et transducteur électrostatique.

La Figure 8 illustre le principe de conversion de fréquence mettant en oeuvre une double structure oscillante et un couplage électrostatique à relaxation.

La Figure 9 illustre les déplacements respectifs des deux structures oscillantes de la configuration de la Figure 8, au cours d'un cycle de sollicitation mécanique de la structure primaire.

La Figure 10 est homologue de la Figure 8, pour une variante de réalisation avec un jeu d'électrodes fixes solidaires du corps de la capsule.

Les Figures 11 à 13 illustrent divers exemples possibles de configuration électrostatique entre les deux structures oscillantes.

Les Figures 14 à 16 illustrent divers exemples possibles de transducteurs.

La Figure 17 illustre un exemple du système de conversion de fréquence utilisant la rotation de doigts électrostatiques rigides par rapport à une structure centrale formant la structure oscillante primaire.

La Figure 18 est une courbe montrant les variations de capacité au cours du temps d'une structure telle que celle de la Figure 17.

La Figure 19 est une courbe montrant les variations de l'énergie générée en sortie au moyen de la structure de la Figure 17, et en comparaison l'énergie générée par un dispositif récupérateur conventionnel.

La Figure 20 illustre un autre exemple du système de conversion de fréquence utilisant la déformation de doigts électrostatiques flexibles sous l'effet de forces d'attraction électrostatique.

La Figure 21 illustre une variante du système de la Figure 20, dans laquelle les extrémités des doigts flexibles sont reliées ensemble afin de s'assurer de la synchronisation de leurs mouvements à haute fréquence.

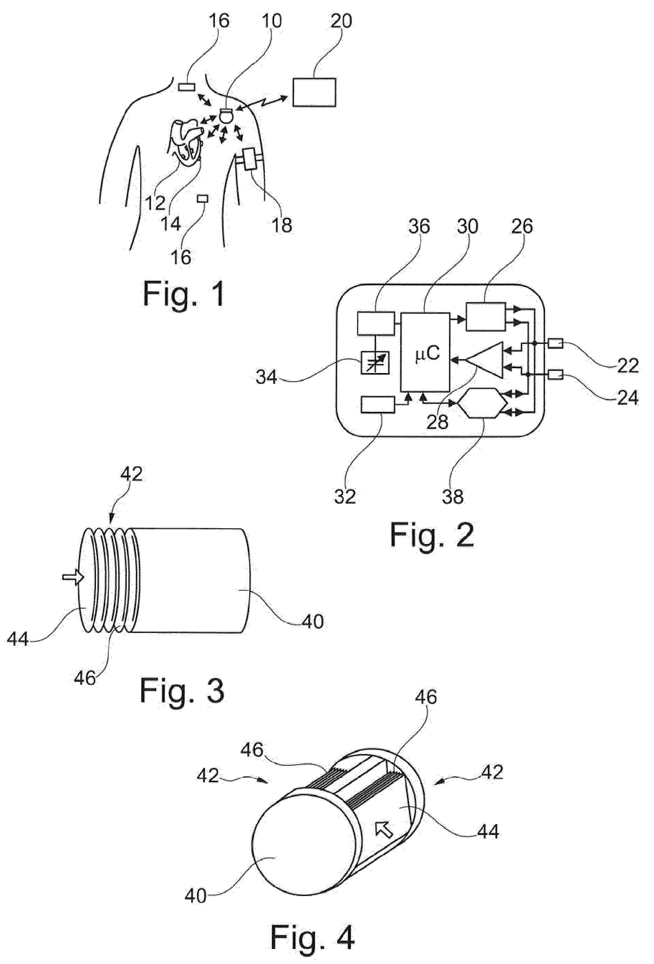

On va tout d'abord décrire, en référence aux Figures 1 à 7, la structure de base d'une capsule *leadless* et un exemple de réalisation d'un récupérateur d'énergie pour une telle capsule, selon l'état de la technique.

Sur la Figure 1 on a illustré un ensemble de dispositifs médicaux implantés au sein du corps d'un patient.

Celui-ci est équipé par exemple d'un implant 10 tel qu'un défibrillateur/ stimulateur/resynchroniseur implanté, un défibrillateur sous-cutané ou encore un enregistreur longue durée. Ce dispositif 10 est le dispositif maitre d'un réseau comportant une pluralité de dispositifs esclaves 12 à 18, qui peuvent notamment inclure des capsules intracardiaques 12 ou épicardiques 14 implantées directement sur le coeur du patient, d'autres dispositifs 16 tels que capteurs de myopotentiels ou dispositifs de stimulation neurologique, et éventuellement un dispositif externe 18 disposé sur un brassard et pourvu d'électrodes en contact avec la peau. Le dispositif 10 peut également être utilisé en tant que passerelle avec le monde extérieur pour communiquer avec un périphérique externe 20 de type programmateur ou dispositif de télétransmission de données avec lequel il pourra communiquer par télémétrie.

La Figure 2 illustre de façon schématique les différents circuits internes des capsules autonomes implantées 12 à 16.

La capsule comporte par exemple un couple d'électrodes 22, 24 reliées à un circuit 26 générateur d'impulsions de stimulation (pour une capsule active incorporant cette fonction) et/ou à un circuit de détection 28 servant au recueil des potentiels de dépolarisation recueillis entre les électrodes 22 et 24. Un circuit central 30 inclut l'ensemble de l'électronique permettant de piloter les diverses fonctions de la capsule, la mémorisation des signaux recueillis, etc. Il comprend un microcontrôleur et un oscillateur générant les signaux d'horloge nécessaires au fonctionnement du microcontrôleur et à la communication. Il peut également contenir un convertisseur analogique/numérique et une mémoire de stockage numérique. La capsule peut également être pourvue d'un capteur 32 tel qu'un capteur d'accélération, de pression, un capteur hémodynamique, de température, de saturation en oxygène, etc. La capsule comprend un module de récupération d'énergie 34 alimentant l'ensemble des circuits via un étage de gestion d'énergie 36. Les électrodes 22 et 24 sont également reliées à un circuit d'émission/réception d'impulsions 38 servant à la communication sans fil avec le dispositif maitre ou les autres capsules.

L'invention concerne plus particulièrement le module de récupération d'énergie 34.

Il s'agit de récupérer l'énergie contenue dans des efforts mécaniques auxquels est soumise la capsule.

Deux cas particuliers de sollicitations extérieures peuvent être envisagés :

- récupération de l'énergie vibratoire, également dénommée énergie inertielle, au moyen d'une masse sismique qui subit les efforts d'accélération et produit une force égale au produit de cette accélération par la masse en mouvement ; ou
- récupération des variations de pression cardiaque, en prévoyant qu'une partie du corps de la capsule soit déformable sous l'effet de ces variations de pression. La force produite est alors proportionnelle au produit de l'amplitude des variations de pression par la surface de l'élément déformable.

Dans l'un ou l'autre cas, la fréquence des sollicitations cycliques se situe, dans le milieu cardiaque, dans des plages de l'ordre de 1 à 3 Hz (fréquence des battements cardiaques) et également autour de 15 à 20 Hz, pour les vibrations d'une masse sismique, et dans la plage 1 à 3 Hz des battements cardiaques pour les variations de la pression sanguine.

Les Figures 3 à 7 illustrent divers exemples adaptés à la récupération des efforts de pression par une capsule telle que divulguée par exemple dans le EP 2 520 333 A1 (Sorin CRM).

Pour prendre en compte les variations de pression, la capsule est réalisée, comme illustré Figures 3 et 4, avec un corps 40 pourvu d'un ou plusieurs éléments déformables 42 sollicités au rythme des variations de la pression du fluide dans lequel baigne la capsule, typiquement les variations de pression sanguine. L'élément déformable 42 comprend une surface rigide 44 sur laquelle s'exercent les variations de pression, et qui est reliée au reste du corps 40 par un soufflet déformable 46. Dans l'exemple de la Figure 3, cet ensemble surface/soufflet 44/46 est disposé sur une face d'extrémité axiale de la capsule 40, tandis que dans l'exemple de la Figure 4 il est prévu deux ensembles surface/soufflet 44/46 disposés sur des faces latérales du corps 40 de la capsule, les surfaces rigides 44 étant parallèles entre elles et à l'axe principal de la capsule.

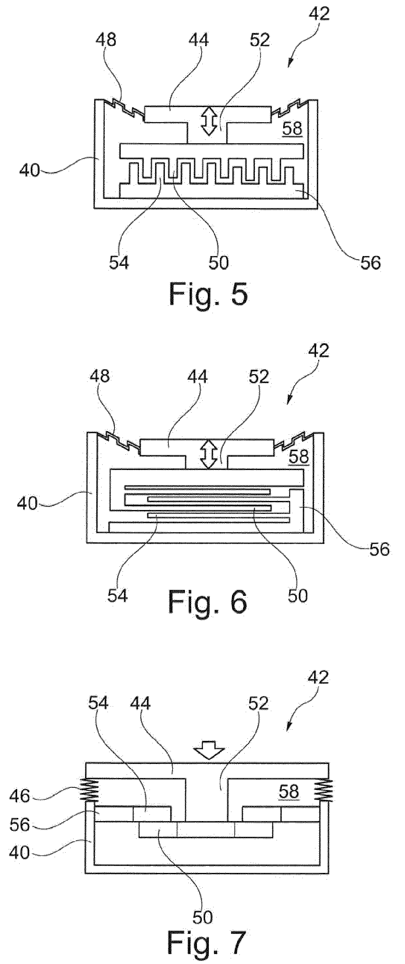

Les Figures 5 à 7 illustrent diverses structures possibles de transducteur électrostatique pour la récupération de l'énergie des variations de pression par l'élément déformable 42. Ce mode de transduction n'est cependant pas limitatif de la présente invention qui, comme on le verra plus loin, peut également mettre en oeuvre un transducteur fonctionnant selon un autre principe, par exemple électromagnétique ou piézoélectrique.

Sur les Figures 5 à 7, la surface mobile 44 est couplée au corps 40 par un élément élastique de liaison 48 (Figures 5 et 6) ou un soufflet 46 (Figure 7). Cette surface mobile 44 est reliée à une série de premières électrodes de conversion 50 via un élément de couplage 52. Ces électrodes sont avantageusement configurées sous la forme de peignes, réalisés par exemple par des techniques classiques de photogravure. Le dispositif comporte par ailleurs des secondes électrodes de conversion 54 réalisées sous la forme de contre-peignes interdigités avec les peignes des électrodes 50 et reliés au corps 40 par un support périphérique 56. L'ensemble formé par ces électrodes 50, 54 est enfermé dans le volume étanche 58 constitué par le corps 40 enfermé par l'élément déformable 42.

On dispose ainsi d'un transducteur qui peut être modélisé par un condensateur à capacité variable comprenant :

- une première électrode de conversion, suspendue, constituée par les peignes 50 qui sont mécaniquement et électriquement réunis entre eux et à la surface mobile via l'élément 52 ;
- une deuxième électrode de conversion, fixe, constituée par les contre-peignes 54 qui sont mécaniquement et électriquement réunis entre eux et au corps 40 via le support 56 ; et
- un intervalle diélectrique, défini entre les deux électrodes.

Dans la configuration de la Figure 5, en cas d'enfoncement de la surface mobile 44 l'entrefer et le chevauchement dans le plan des peignes restent constants, mais le chevauchement vertical change au cours du mouvement. Dans le cas de la Figure 6, les électrodes sont configurées avec un intervalle diélectrique variable, tandis que dans le cas de la Figure 7 les électrodes sont configurées avec chevauchement dans le plan, le reste de la structure étant identique à ce qui a pu être décrit à la Figure 5. Mais dans tous les cas, on dispose d'un élément dont la capacité varie cycliquement au rythme des déplacements de la surface mobile 44 et donc des variations de la pression du milieu sanguin environnant.

Si le condensateur a été chargé au préalable, une diminution de sa capacité produira un surcroit d'énergie qui pourra être déchargé par des circuits appropriés vers un module de stockage, et *vice versa.* À chaque cycle cardiaque on pourra ainsi récupérer une quantité d'énergie qui sera à terme suffisante pour assurer un fonctionnement permanent des circuits électroniques de la capsule, sans apport supplémentaire d'énergie.

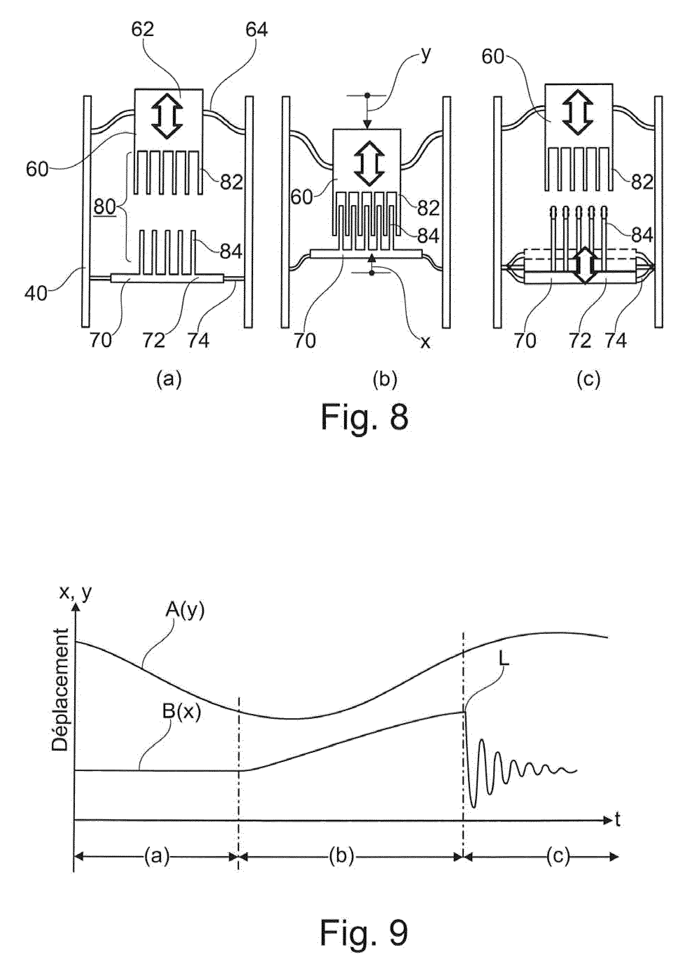

On va maintenant décrire, en référence aux Figures 8 à 21, le perfectionnement permettant d'augmenter très substantiellement le rendement de conversion des dispositifs connus tels que ceux dont on vient de décrire le fonctionnement de base.

La Figure 8 illustre ce principe de conversion. Essentiellement, il s'agit d'utiliser les forces électrostatiques mises en jeu lors de l'application d'une tension d'une charge électrique entre deux parties d'un condensateur à capacité variable afin de provoquer un phénomène de "tirer-lâcher" d'une structure de transduction d'énergie mécanique en énergie électrique.

Concrètement, cette structure comprend deux parties mobiles, ci-après désignées "structure oscillante primaire" (désignée 60 sur les figures) et "structure oscillante secondaire" (désignée 70 sur les figures, ces références numériques étant conservées sur toutes les figures pour désigner, des structures fonctionnellement semblables).

La structure primaire 60 est soumise à une sollicitation extérieure à basse fréquence telle qu'une force directe ou, comme dans le mode de réalisation de la Figure 8, aux vibrations d'une masse sismique 62 montée sur une suspension élastique 64 reliée au corps 40 de la capsule.

La structure secondaire 70, quant à elle, comprend un élément 72 mobile et un élément élastiquement déformable 74, qui est dans le mode de réalisation de la Figure 8 une suspension reliant la partie mobile 70 au corps 40 de la capsule.

La masse de la partie mobile 72 et l'élasticité de la suspension 74 définissent pour la structure secondaire 70 une fréquence de résonance propre, qui de façon caractéristique est une fréquence notablement plus élevée que la fréquence à laquelle est sollicitée la structure primaire 60.

Les deux structures oscillantes 60, 70 sont couplées entre elles par une structure électrostatique référencée 80, constituée de deux séries respectives d'électrodes 82, 84 dont le déplacement mutuel a pour effet de faire varier la capacité du condensateur constitué par ces électrodes respectives. On exposera ci-après diverses configurations possibles de ces électrodes 82, 84 de la structure électrostatique 80, les mêmes références numériques 80, 82 et 84 étant conservées sur les différentes figures pour désigner des éléments fonctionnellement semblables.

Dans la Figure 8, les électrodes 82 sont solidaires de la structure oscillante primaire 60, et les électrodes 84 sont solidaires de la structure oscillante secondaire 70.

On va maintenant exposer le comportement dynamique de cette structure, en référence aux Figures 8 et 9.

La structure primaire 60, lors de son déplacement (à la fréquence de la sollicitation extérieure cyclique, correspondant à la courbe A de la Figure 9) va se retrouver en regard de la structure secondaire 7 avec imbrication progressive des électrodes 82, 84 (passage de l'état (a) à l'état (b) de la Figure 8).

Si l'on applique une tension ou une charge électrique entre les électrodes 82, 84 en regard, une interaction électrostatique, typiquement une attraction, a lieu entre ces électrodes. Cette interaction aura pour effet d'attirer la structure secondaire 70 vers la structure primaire 60, comme illustré par la courbe B de la Figure 9, qui représente le déplacement de la structure 70 : l'attraction résultant de l'interaction électrostatique correspond au déplacement dans la région (b) de la Figure 9.

La force d'attraction électrostatique entre les deux structures 60 et 70 va passer par un maximum, suffisamment fort pour que la structure secondaire 70 soit entraînée par la structure primaire 60 (étape de "tirer"). Mais la course de la structure secondaire 70 étant plus limitée, une fois une limite L atteinte la rigidité de la suspension 74 va séparer les deux structures (étape de "lâcher").

N'étant plus attirée par la structure primaire 60 qui s'éloigne, la structure secondaire 70, lâchée à partir d'un état étiré de la suspension 74, va entrer en vibration libre à sa fréquence de résonance propre, comme illustré en (c) sur les Figures 8 et 9.

La structure secondaire 70 étant celle qui assure la transduction, ces vibrations vont entrainer de nombreux cycles de transduction avant que la structure primaire ne revienne dans la zone d'action de la structure secondaire, pour effectuer un nouveau cycle-à-cycle de tirer-lâcher.

Pour que l'effet recherché de "tirer-lâcher" soit significatif, il faut que l'attraction électrostatique engendre une déformation du même ordre de grandeur que les dimensions caractéristiques de la structure.

Ici et dans la suite, l'expression "du même ordre de grandeur" devra être comprise dans son acception spécifique conventionnellement employée en physique, c'est-à-dire "dans un rapport de 0,1 à 10".

Plus concrètement, la force électrostatique d'attraction $f_e$ (exprimée en N) entre les doigts formant les électrodes est :

$$f_e = \varepsilon * L * V^2 / g$$

$\varepsilon$ étant la permittivité du gaz (typiquement de l'air ou le vide) entre les doigts formant les électrodes du condensateur,

L étant la longueur de la surface en regard des électrodes dans la dimension perpendiculaire au déplacement de couplage,

V étant la tension électrique appliquée entre les électrodes, et

g étant l'entrefer séparant ces électrodes.

Si l'élément déformable soutenant la structure secondaire a une raideur $k$ (en N/m), alors la force élastique de rappel $f_r$ sous une déformation x est (en N) :

$$f_r = k * x.$$

Ainsi, sous équilibre quasi-statique lors du mouvement de tirer, le déplacement est :

$$x = \varepsilon * L * V^2 / (g * k),$$

Si l'on note h une dimension caractéristique des doigts, comme par exemple leur épaisseur, alors pour avoir un déplacement x du même ordre de grandeur que h, il faut une raideur $k$ qui soit au moins du même ordre de grandeur que $\varepsilon * L * V^2 / (g * h)$.

*A contrario,* l'élément déformable ne doit pas être trop souple, c'est-à-dire avec *k* trop faible, car sinon il n'y aura jamais de "lâcher", la raideur n'étant jamais assez importante pour faire sortir la structure de l'attraction électrostatique et produire la relaxation autour de la position d'équilibre.

C'est pourquoi il faut que la raideur *k* du système soit égale (à un ordre de grandeur près) à $\varepsilon^*L^*V^2/(g^*h)$.

Du point de vue mécanique, si l'on assimile chaque doigt à une poutre de longueur L, d'épaisseur h (dans le sens de la direction de flexion) et de largeur w (perpendiculairement à la direction de flexion), on peut exprimer la valeur de la force électrostatique comme une force linéique (exprimée en N/m) de valeur $f_e = \varepsilon^*V^2/g$, s'exerçant le long des doigts.

En utilisant les lois de la mécanique, on peut exprimer la rigidité $f_r$ du doigt par unité de longueur (en N/m), lorsque ce dernier subit une flexion d'amplitude x en bout de doigt, par :

$$f_r = 2/3^*E^*x^*h^{3*}w/L^4$$

où E représente le module d'Young du matériau du doigt.

Le phénomène de flexion des doigts, et donc de conversion de fréquence, devient significatif lorsque $f_e$ devient notablement supérieure à $f_r$, pour une déflexion x de l'ordre de grandeur de *h.*

Ainsi, pour produire les effets caractéristiques de la présente invention, les doigts doivent être dimensionnés de telle manière que la valeur de $\varepsilon^*V^2/g$ soit égale à celle de $E^*x^*h^{3*}w/L^4$, à un ordre de grandeur près.

En outre, pour éviter les instabilités des doigts dans le plan, à savoir le fait qu'ils peuvent se défléchir dans le plan en réduisant l'entrefer (effet *pullin*), les doigts ont préférablement une largeur w plus grande que leur épaisseur h, pour les rendre rigides dans le plan, comparativement à leur flexibilité hors-plan souhaitée.

La multiplication de la fréquence de fonctionnement du transducteur (à la fréquence propre de résonance de la structure oscillante secondaire 70 et non à la fréquence basse des sollicitations externes cycliques) a pour effet d'augmenter notablement le rendement de conversion des transducteurs transformant l'énergie mécanique des efforts appliqués à la capsule en énergie électrique. En effet, si à chaque cycle de transduction le transducteur convertit une fraction donnée de l'énergie mécanique en énergie électrique, et si la fréquence de transduction est un multiple de la fréquence d'excitation, alors le rendement de transduction par cycle d'excitation est multiplié d'autant.

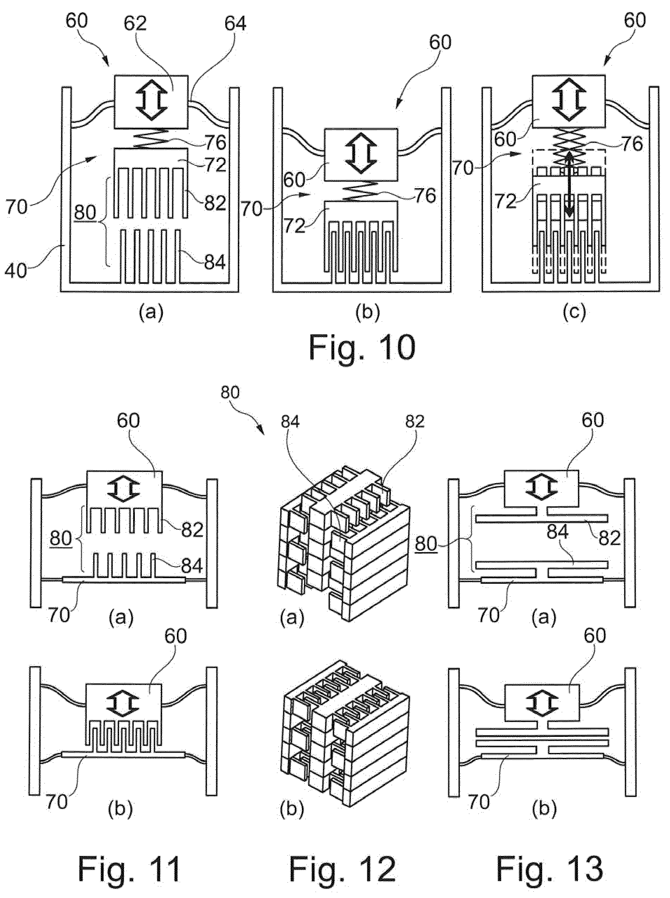

Dans le cas de la Figure 10, l'élément élastique déformable qui définit la fréquence de résonance de la structure secondaire 70 n'est plus, comme dans la Figure 8, une suspension couplant cette structure au corps de la capsule, mais un ressort 76 reliant la masse sismique 62 de la structure primaire 60 à la partie mobile 72 de la structure secondaire 70. Les électrodes 82 sont toujours portées par la partie mobile 72 de la structure secondaire 70, en revanche les électrodes 84 sont des électrodes fixes solidaires du corps 40 de la capsule.

Comme illustré en (a) sur la Figure 10, le système combinant la structure primaire 60 et la structure secondaire 70 a initialement un mouvement uniforme induit par l'excitation à basse fréquence de la structure primaire 60 par la sollicitation extérieure. Lorsque cette structure entre dans le domaine d'attraction électrostatique de la structure fixe constituée par les électrodes 84 comme illustré à l'étape (b) de la Figure 10, alors la structure secondaire 70 va subir, en plus de sa force de rappel la liant à la structure primaire 60 (du fait du ressort 76), une force d'attraction électrostatique qui va provoquer une dissociation du mouvement des deux structures primaire 60 et secondaire 70 (étape de "tirer"). De même que pour la Figure 8, lorsque la structure primaire 60, dans son mouvement d'oscillation lente, commence à s'éloigner, la rigidité entre les deux structures l'emporte (étape de "lâcher") et la structure secondaire 70 sort brutalement du domaine d'attraction de la structure électrostatique fixe 82, provoquant la vibration à haute fréquence avec multiplication des cycles de transduction, comme illustré en (c) sur la Figure 10.

Les Figures 11, 12 et 13 illustrent trois exemples possibles de structure électrostatique, où la configuration des surfaces en regard comprend avantageusement de larges surfaces séparées par un faible entrefer, de manière à créer une forte capacité et donc une forte variation de cette capacité lors du déplacement relatif des électrodes. On peut ainsi prévoir notamment des peignes interdigités pour maximiser les surfaces en regard, avec un chevauchement variable, hors plan (Figure 11) ou dans le plan (Figure 12). La variation de capacité peut également résulter d'une variation d'entrefer entre les surfaces en regard des électrodes (Figure 13).

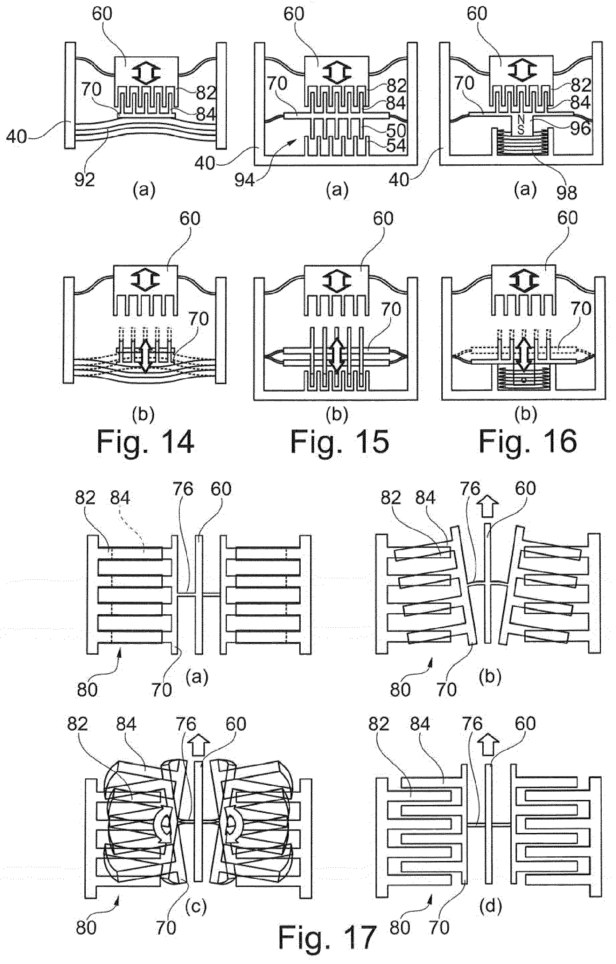

La transduction en énergie électrique du mouvement de la structure secondaire 70 peut être opérée de diverses manières, illustrées schématiquement Figures 14 à 16.

La Figure 14 illustre le cas d'un transducteur piézoélectrique, par exemple en prévoyant de réaliser en partie la suspension de la structure secondaire 70 portant les électrodes 84 avec un matériau piézoélectrique 92, qui va subir des contraintes lors du déplacement et va donc générer des charges sur ces électrodes. Ces électrodes sont situées de part et d'autre du matériau piézoélectrique 92 et sont reliées à un circuit électronique de gestion, typiquement un redresseur de courant et un condensateur de filtrage.

La Figure 15 illustre le cas d'un transducteur électrostatique, qui comprend des électrodes 50, 54 (fonctionnellement comparables à celles décrites plus haut à propos des Figures 5 à 7) formant un condensateur 94 dont la capacité varie à fréquence élevée. Les bornes de ce condensateur 94 sont reliées à un circuit électronique effectuant à chaque cycle de variation de capacité une charge et une décharge du condensateur, de façon à générer une énergie électrique positive. On notera que sur la Figure 15 on a décrit un transducteur 94 comprenant un jeu d'électrodes 50, 54 distinctes des électrodes 82, 84 de la structure électrostatique 80 réalisant la conversion de fréquence selon l'invention. Il est toutefois possible, et même avantageux, de mettre en oeuvre la transduction d'énergie électrique au moyen du condensateur variable constitué par les électrodes 82, 84 de la structure électrostatique 80 en d'autres termes d'utiliser ces électrodes 82, 84 non seulement pour le couplage des structures oscillantes et la conversion de fréquence d'oscillation (fonction mécanique), mais également pour assurer la conversion du mouvement en énergie électrique (fonction de transduction mécanoélectrique).

La Figure 16 illustre le cas d'un transducteur électromagnétique, dans lequel la partie mobile de la structure secondaire 70 supporte un aimant permanent 96 mobile dans une bobine fixe 98 solidaire du corps 40 (ou *vice versa)* de manière à générer un champ magnétique variable à haute fréquence.

Ces divers types de transducteurs ne sont pas limitatifs, et d'autres techniques peuvent être également utilisées, par exemple à base de transducteurs magnétostrictifs, utilisant des polymères électroactifs, etc.

La Figure 17 illustre un autre exemple du système de conversion de fréquence mettant en oeuvre une rotation des électrodes 84 par rapport aux électrodes 82, fixes et rigides.

Les états (a) à (d) illustrent les phases successives de déformation et des déplacements relatifs des différents éléments de l'ensemble : au repos (état (a)), les peignes et contre-peignes des électrodes respectives 82, 84 sont immobiles et face à face. L'élément central 60 de l'ensemble constitue la structure primaire, qui est soumise aux forces extérieures, via une masse sismique dans le cas d'une récupération inertielle, ou d'une surface mobile avec un soufflet dans le cas d'une récupération des variations de pression, et son mouvement est donc dicté par cette sollicitation extérieure cyclique à basse fréquence. Au début du mouvement (état (b) sur la Figure 17), la partie centrale 60 commence à se décaler, cependant les peignes 84 sont soumis à une attraction électrostatique des contre-peignes 82, qui s'oppose au mouvement. De la sorte, l'élément flexible 76 reliant l'élément central 60 impose aux peignes et contre-peignes 82, 84 de rester au maximum en regard les uns des autres (étape de "tirer"). À mesure que le mouvement s'amplifie, la rigidité de l'élément flexible 76 l'emporte sur l'attraction électrostatique, et les peignes vont être subitement relâchés (étape de "lâcher" correspondant à l'état (c) sur la Figure 17). Ces peignes 84 vont alors entrer dans une phase de vibration libre à haute fréquence, cette fréquence qui peut atteindre plusieurs centaines voire milliers de hertz étant définie par la masse, très faible, des peignes 84 et la raideur de l'élément flexible 76. Lors de cette vibration, la capacité entre les peignes 84 et les contre-peignes 82 va également varier à haute fréquence, et de nombreux cycles de charge/décharge du condensateur vont pouvoir être effectués, augmentant grandement l'énergie extraite par unité de temps.

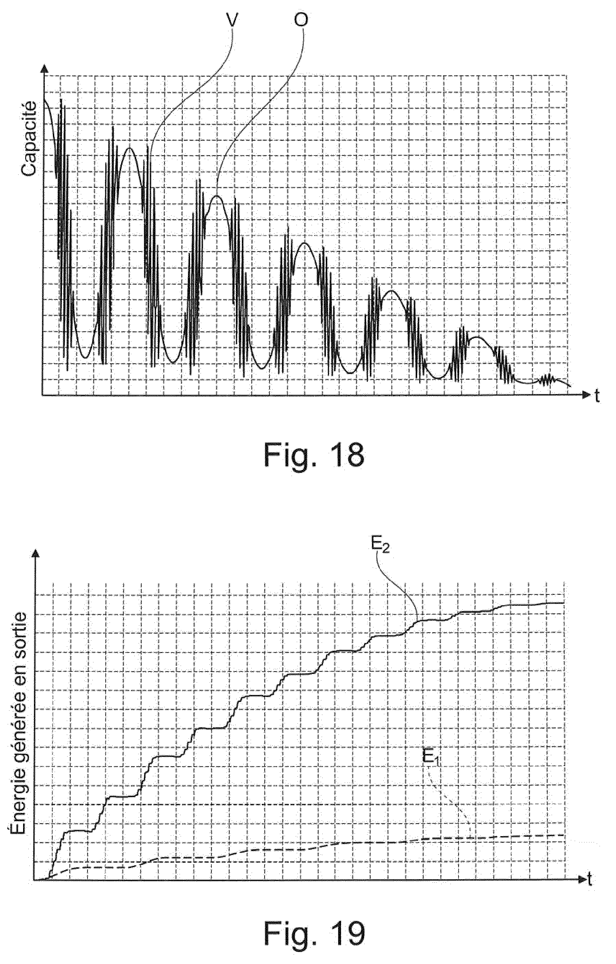

La Figure 18 illustre les variations au cours du temps de la capacité du condensateur constitué par les peignes et contre-peignes 82 et 84 de l'ensemble de la Figure 17. La Figure 19 illustre l'énergie récupérée en sortie grâce à cette variation de capacité.

Ces Figures 18 et 19 illustrent des courbes relevées pour le système de la Figure 17, mais ces variations de capacité et d'énergie seraient obtenues de façon semblable avec les autres structures exposées dans la présente description.

Sur la Figure 18, on voit que la variation de capacité comprend une sinusoïde d'amplitude décroissante O superposée à des variations à haute fréquence V. Grâce au phénomène de conversion de fréquence, le condensateur variable va présenter un très grand nombre de maxima et minima par unité de temps ce qui, pour un même cycle de sollicitation, va permettre, comme on peut le voir sur la Figure 19, de récupérer une énergie électrique $E_2$ bien plus élevée que celle $E_1$ qui aurait été obtenue avec un dispositif conventionnel sans conversion de fréquence.

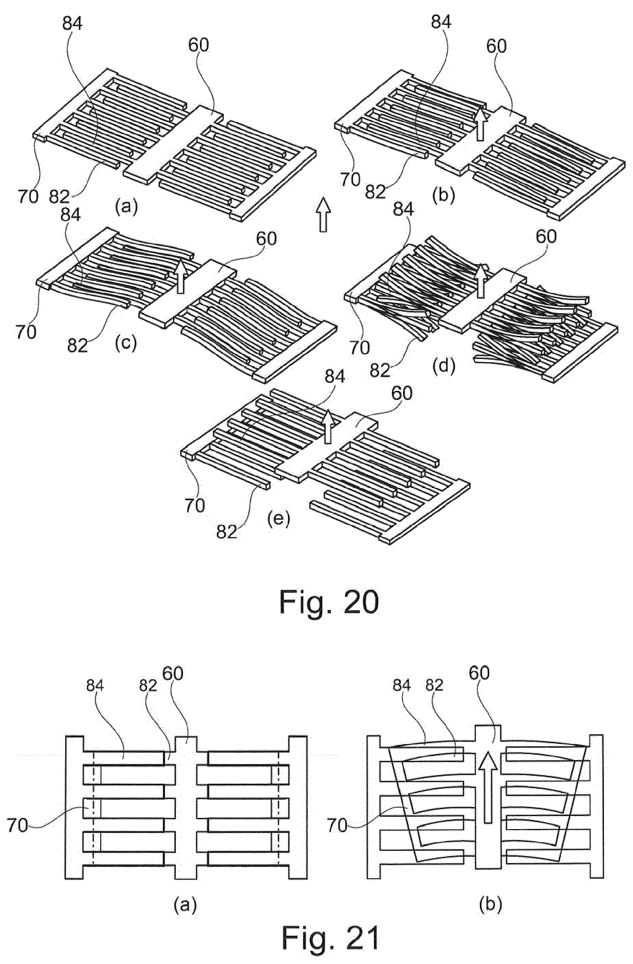

La Figure 20 décrit une variante dans laquelle les doigts électrostatiques 82, 84 de la structure électrostatique 80 constituent l'élément élastique déformable définissant la fréquence de résonance propre de la structure oscillante secondaire 70.

Plus précisément, les doigts 82, 84 des peignes et contre-peignes présentent une géométrie telle que des phénomènes d'instabilité électrostatique au niveau de ces doigts se produisent lors du déplacement de la partie mobile, créant ainsi des variations de capacité à haute fréquence. Les doigts présentent à cet effet un rapport d'aspect élevé, avec une forme allongée et peu épaisse, d'où une grande flexibilité hors plan permettant aux forces électrostatiques verticales d'être significatives.

Comme illustré par les différents états (a) à (e) sur la Figure 20, lors du déplacement vertical de la partie mobile 60 les doigts 84 des peignes vont se plier de façon à rester en regard des doigts 82 des contre-peignes, la force d'attraction électrostatique peignes/contre-peignes étant forte devant la rigidité mécanique des peignes. Cependant, lorsque le déplacement des peignes grandit, la rigidité des peignes reprend le dessus et les peignes sont libérés spontanément de leur attraction électrostatique vis-à-vis des contre-peignes. Si l'on superpose plusieurs couches de peignes/contre-peignes tels que ceux illustrés Figure 20, lorsque les peignes seront libérés de l'attraction des contre-peignes de la couche initiale, ils vont rentrer dans le champ d'attraction des contre-peignes de la couche supérieure.

Ce saut d'une couche à l'autre va provoquer une vibration à haute fréquence des peignes, engendrant une variation de capacité du transducteur à cette même fréquence et augmentant ainsi le nombre de cycles de charge et de décharge du condensateur, et augmentant au final l'énergie récupérée.

Comme illustré Figure 21, il peut être intéressant de coupler ensemble les extrémités des doigts 84 de manière à permettre une synchronisation parfaite de leurs déplacements sur les différentes couches, et donc de la variation de capacité issue de chaque doigt.

## Revendications

1. Une capsule intracorporelle autonome comportant un corps (40) et, à l'intérieur de ce corps, des circuits électroniques (26-38) et un module de récupération d'énergie (34) pour l'alimentation électrique de ces circuits électroniques,
   le module de récupération d'énergie (34) comprenant un transducteur apte à convertir en énergie électrique une sollicitation physique extérieure cyclique appliquée au corps de la capsule et résultant de variations de pression dans le milieu environnant la capsule et/ou de mouvements d'une paroi à laquelle est ancrée la capsule,
   dans laquelle le module de récupération d'énergie comprend :

   - une structure oscillante primaire (60) soumise à ladite sollicitation cyclique extérieure, cette structure oscillante primaire étant apte à être déplacée alternativement dans un sens et dans l'autre à la fréquence de ladite sollicitation cyclique extérieure ;
   - une structure oscillante secondaire (70) non soumise à ladite sollicitation extérieure cyclique, cette structure oscillante secondaire comprenant un élément élastique déformable (76) et étant apte à vibrer librement à une fréquence de résonance propre supérieure à ladite fréquence de la sollicitation cyclique extérieure ; et
   - une structure électrostatique (80) comprenant une première électrode de condensateur (84) et une seconde électrode de condensateur (82) couplée à la structure oscillante secondaire (70),

   dans laquelle la première et la seconde électrode sont configurées entre elles de manière que, sous l'effet de ladite sollicitation cyclique extérieure appliquée à la structure oscillante primaire, elles exercent entre elles sous l'effet d'interactions électrostatiques une force d'attraction mutuelle entrainant la structure oscillante secondaire en éloignement de sa position d'équilibre stable et avec mise en tension de l'élément élastique déformable,
   la structure oscillante secondaire étant ainsi entrainée jusqu'à une limite (L) où la tension exercée par l'élément élastique déformable dépasse la force d'attraction mutuelle des électrodes,
   limite au-delà de laquelle la structure oscillante secondaire est libérée par effet de relaxation pour vibrer librement à ladite fréquence de résonance propre,
   et dans laquelle le transducteur est couplé à la structure oscillante secondaire, pour convertir en énergie électrique les mouvements de vibration de celle-ci à ladite fréquence de résonance propre,
   **caractérisée en ce que** :

   - la structure oscillante primaire (60) et la structure oscillante secondaire (70) sont couplées entre elles par ledit élément élastique déformable (76) ; et
   - la première électrode (84) de la structure électrostatique (80) est reliée au corps (40) de la capsule.

2. La capsule de la revendication 1, dans laquelle le transducteur est de type électrostatique comprenant des électrodes de conversion (82, 84) formant un condensateur, l'une de ces électrodes de conversion étant entrainée cycliquement par la structure oscillante secondaire (70), et le transducteur opérant par modification cyclique des surfaces en vis-à-vis et/ou de l'intervalle diélectrique des électrodes de conversion avec variation corrélative de la capacité du condensateur.

3. La capsule de la revendication 2, dans laquelle lesdites électrodes de conversion incluent lesdites première et seconde électrodes (82, 84) de la structure électrostatique (80).

## Patentansprüche

1. Autonome intrakorporelle Kapsel, die einen Körper (40) und im Inneren dieses Körpers elektronische Schaltkreise (26-38) und ein Energierückgewinnungsmodul (34) für die Stromversorgung dieser elektronischen Schaltkreise enthält,
   wobei das Energierückgewinnungsmodul (34) einen Wandler enthält, der geeignet ist, eine äußere zyklische physikalische Beanspruchung, die auf den Körper der Kapsel ausgeübt wird und aus den Druckschwankungen in der Umgebung der Kapsel und/oder aus den Bewegungen einer Wand, an der die Kapsel verankert ist, resultiert, in elektrische Energie umzuwandeln,
   bei der das Energierückgewinnungsmodul Folgendes enthält:

   - eine primäre oszillierende Struktur (60), die der genannten äußeren zyklischen Beanspruchung ausgesetzt ist, wobei diese primäre oszillierende Struktur geeignet ist, nach der Frequenz der genannten äußeren zyklischen Beanspruchung abwechselnd in die eine und die andere Richtung verschoben zu werden;
- eine sekundäre oszillierende Struktur (70), die der genannten äußeren zyklischen Beanspruchung nicht ausgesetzt ist, wobei diese sekundäre oszillierende Struktur ein verformbares elastisches Element (76) enthält, das fähig ist, frei mit einer Eigenresonanzfrequenz zu schwingen, die größer ist als die genannte Frequenz der äußeren zyklischen Beanspruchung; und
- eine elektrostatische Struktur (80), die eine erste Kondensatorelektrode (84) und eine zweite Kondensatorelektrode (82) enthält und mit der sekundären oszillierenden Struktur (70) gekoppelt ist,

in welcher die erste und die zweite Elektrode untereinander so konfiguriert sind, dass sie unter dem Einfluss der genannten äußeren zyklischen Beanspruchung, die auf die primäre oszillierende Struktur ausgeübt wird, auf Grund von elektrostatischen Wechselwirkungen eine gegenseitige Anziehungskraft aufeinander ausüben, wodurch die sekundäre oszillierende Struktur aus ihrer stabilen Gleichgewichtslage heraus- und mitgeführt wird und das verformbare elastische Element unter Spannung gesetzt wird,
wobei die sekundäre oszillierende Struktur dabei bis zu einem Grenzwert (L) mitgeführt wird, an dem die durch das verformbare elastische Element ausgeübte Spannung die gegenseitige Anziehungskraft der Elektroden überschreitet, und über dem die sekundäre oszillierende Struktur durch den Entspannungseffekt freigegeben wird und anschließend frei mit der genannten Eigenresonanzfrequenz schwingt,
und in welcher der Wandler mit der sekundären oszillierenden Struktur gekoppelt ist, um deren Schwingungsbewegungen bei der genannten Eigenresonanzfrequenz in elektrische Energie umzuwandeln, **dadurch gekennzeichnet, dass**

- die primäre oszillierende Struktur (60) und die sekundäre oszillierende Struktur (70) durch das genannte verformbare elastische Element (76) miteinander verbunden sind; und
- die erste Elektrode (84) der elektrostatischen Struktur (80) mit dem Körper (40) der Kapsel verbunden ist.

2. Kapsel nach Anspruch 1, in welcher der Wandler elektrostatischen Typs ist und Wandlerelektroden (82, 84) enthält, die einen Kondensator bilden, wobei eine dieser Wandlerelektroden durch die sekundäre oszillierende Struktur (70) zyklisch mitgeführt wird, und der Wandler durch die zyklische Veränderung der gegenüberliegenden Flächen und/oder des dielektrischen Abstands der Wandlerelektroden mit damit verbundener Veränderung der Kapazität des Kondensators funktioniert.

3. Kapsel nach Anspruch 2, in der die genannten Wandlerelektroden die genannte erste und zweite Elektrode (82, 84) der elektrostatischen Struktur (80) enthalten.

**Claims**

1. An autonomous intracorporeal capsule including a body (40) and, inside this body, electronic circuits (26-38) and an energy harvesting module (34) for the power supply of these electronic circuits,
the energy harvesting module (34) comprising a transducer adapted to convert into electric energy a cyclic external physical stress applied to the capsule body and resulting from pressure variations in the medium surrounding the capsule and/or from movements of a wall to which the capsule is anchored, wherein the energy harvesting module comprises:

- a primary oscillating structure (60) subjected to said external cyclic stress, this primary oscillating structure being adapted to be alternately moved in one direction and in the other at the frequency of said external cyclic stress;
- a secondary oscillating structure (70) non subjected to said cyclic external stress, this secondary oscillating structure comprising a deformable elastic element (76) and being adapted to vibrate freely at a self-resonant frequency higher than said frequency of the external cyclic stress; and
- an electrostatic structure (80) comprising a first capacitor electrode (84) and a second capacitor electrode (82) coupled to the secondary oscillating structure (70),

wherein the first and the second electrode are configured between each other so that, under the effect of said external cyclic stress applied to the primary oscillating structure, they exert between each other, under the effect of electrostatic interactions, a force of mutual attraction driving the secondary oscillating structure away from its stable equilibrium position and with tensioning of the deformable elastic element,
the secondary oscillating structure being hence driven to a limit (L) where the tension exerted by the deformable elastic element exceeds the force of mutual attraction of the electrodes,
limit beyond which the secondary oscillating structure is released by a relaxation effect to vibrate freely at said self-resonant frequency, and wherein the transducer is coupled to the secondary oscillating structure, to convert into electric energy the movements of vibration of the latter at said self-resonant frequency,
**characterized in that**:

- the primary oscillating structure (60) and the secondary oscillating structure (70) are coupled to each other by said deformable elastic element (76); and
- the first electrode (84) of the electrostatic structure (80) is connected to the body (40) of the capsule.

2. The capsule of claim 1, wherein the transducer is of electrostatic type comprising conversion electrodes (82, 84) forming a capacitor, one of these conversion electrodes being cyclically driven by the secondary oscillating structure (70), and the transducer operating by cyclic modification of the opposite surfaces and/or of the dielectric interval of the conversion electrodes with correlative variation of the capacitor's capacitance.

3. The capsule of claim 2, wherein said conversion electrodes include said first and second electrodes (82, 84) of the electrostatic structure (80).

16  10  20

Fig. 1

36  30  26

34

μC

22

24

28

32  38

Fig. 2

42

44

46

Fig. 3

46

46

42

42

44

40

Fig. 4

Fig. 5

Fig. 6

Fig. 7

Fig. 8

Fig. 9

Fig. 10

Fig. 11

Fig. 12

Fig. 13

Fig. 14    Fig. 15    Fig. 16

Fig. 17

Fig. 18

Fig. 19

Fig. 20

Fig. 21

**EP 2 857 065 B1**

## RÉFÉRENCES CITÉES DANS LA DESCRIPTION

**Documents brevets cités dans la description**

- US 20070088397 A1
- WO 2007047681 A2
- US 20060136004 A1
- US 20110140577 A1
- EP 2520333 A
- EP 2520333 A1